# EUROPEAN PATENT SPECIFICATION

(11) **EP 1 706 158 B1**
(45) Date of publication and mention of the grant of the patent: **01.09.2010**
(21) Application number: 05705214.4
(22) Date of filing: 07.01.2005
(51) Int. Cl.: A61L 29/18, A61L 31/18, A61L 29/14, A61L 31/14, A61K 49/18, G01R 33/28

(54) **MEDICAL DEVICES VISIBLE UNDER MAGNETIC RESONANCE IMAGING**
MITTELS KERNSPIN-TOMOGRAPHIE SICHTBARE MEDIZINISCHE GERÄTE
DISPOSITIFS MEDICAUX VISIBLES SOUS IMAGERIE PAR RESONANCE MAGNETIQUE

(30) Priority: 09.01.2004 US 755164
(43) Date of publication of application: 04.10.2006
(73) Proprietor: Boston Scientific Scimed, Inc., Maple Grove, MN 55311 (US)
(72) Inventor: ZHONG, Sheng-Ping, Shrewsbury, MA 01545 (US); MA, Enxin, Framingham, MA 01701 (US); DAO, Kinh-Luan, Randolph, MA 02368 (US)
(74) Representative: Hermann, Gerhard
(86) International application number: PCT/US2005/000449
(87) International publication number: WO 2005/070475

(56) References cited:
- EP-A- 0 963 796
- WO-A-00/50103
- WO-A-03/045457
- WO-A-03/094975
- DATABASE WPI Section Ch, Week 198923 Derwent Publications Ltd., London, GB; Class A96, AN 1989-170402 XP002329043 & JP 01 113059 A (SEKISUI CHEM IND CO LTD) 1 May 1989 (1989-05-01)

## Description

### FIELD OF THE INVENTION

The present invention relates to medical devices that are adapted to be visible under magnetic resonance imaging (MRI). More particularly, the present invention relates to medical devices provided with a coating, which is adapted to render the medical device visible under MRI.

### BACKGROUND OF THE INVENTION

The ability to non-invasively image internal body structures and diseased tissues within a patient's body is an extremely important diagnostic tool in the practice of modern medicine. Such non-invasive imaging techniques include magnetic resonance imaging (MRI), x-ray imaging, ultrasonic imaging, x-ray computed tomography, emission tomography, and others. Magnetic resonance imaging can provide two-dimensional cross-sectional images through a patient, providing color or gray scale contrast images of a portion of the body. These two-dimensional images can then be reconstructed to provide a 3-dimensional image of a portion of the body. MRI is advantageous, *inter alia,* because it does not expose the patient or medical practitioner to harmful radiation and it can provide detailed images of the observed area. These detailed images are valuable diagnostic aids to medical practitioners and can be used, for example, to devise, monitor or alter a treatment approach.

Magnetic resonance imaging (MRI) produces images by differentiating detectable magnetic species in the portion of the body being imaged. In the case of ¹H MRI, the detectable species are protons (hydrogen nuclei) that possess an inherent spin magnetic moment such that these protons behave like tiny magnets. Images are typically obtained by placing an area of interest within a powerful, highly uniform, static magnetic field. The protons in the area of interest align like tiny magnets in this field. Radiofrequency pulses are then utilized to create an oscillating magnetic field perpendicular to the main field, from which the nuclei absorb energy and move out of alignment with the static field in a state of excitation. As the nuclei return from excitation to the equilibrium or relaxed state, a signal induced in the receiver coil of the instrument ' by the nuclear magnetization can then be transformed by a series of algorithms into diagnostic images. Images based on different tissue characteristics can be obtained by varying the number and sequence of pulsed radiofrequency fields in order to take advantage of magnetic relaxation properties of the detectable protons in the area of interest.

The environment of the detectable protons alters their magnetic properties and hence the ability of the MRI device to detect such protons and differentiate them from other protons in the surrounding environment. In order to obtain good images, MRI relies upon the differentiation of such protons to provide contrast between the area of interest and the surrounding environment. For example, diseased or damaged tissue may result in a sufficiently different environment for the detectable protons therein relative to that of protons in the surrounding environment. Sufficient contrast is thereby provided by the inherently different environments to produce a good image of the area of interest.

However, in order to enhance the differentiation of detectable species in the area of interest from those in the surrounding environment, contrast agents are often employed. These agents alter the magnetic environment of the detectable protons in the area of interest relative to that of protons in the surrounding environment, thereby allowing for enhanced contrast and hence better images of the area of interest.

For contrast-enhanced MRI it is desirable that the contrast agent have a large magnetic moment. Based upon these criteria, contrast agents such as Gd(III), Mn(II) and Fe(III) have been employed. Gadolinium(III) has the largest magnetic moment among these three and is, therefore, a widely-used paramagnetic species to enhance contrast in MRI. Chelates of paramagnetic ions such as Gd-DTPA (gadolinium ion chelated with the ligand diethylenetriaminepentaacetic acid) have been employed as MRI contrast agents. Chelation of the gadolinium or other paramagnetic ion is believed to reduce the toxicity of the paramagnetic metal by rendering it more biocompatible, and can assist in localizing the contrast agent in the area of interest.

Implantable or insertable medical devices such as catheters, guidewires, balloons, stents, and a variety of other implantable or insertable medical devices are conventionally used to both diagnose and treat medical conditions. To maximize the effectiveness of such medical devices, it is commonly desirable to both properly position the device within a patient and thereafter ascertain the precise location of such device upon implantation or insertion thereof.

The ability of MRI to produce extremely detailed images of an area of interest, and the minimization of harmful radiation exposure to the patient or medical practitioner are distinct advantages of MRI over other imaging techniques. To this end, MRI has been used with varying degrees of success to assist in the placement of a medical device and/or to determine the position of a medical device upon insertion or implantation. Unfortunately, most implantable or insertable medical devices are composed of materials such as organic polymers, metals, ceramics, or composites thereof, which do not produce adequate signals for detection by MRI techniques.

The present invention is, therefore, directed to implantable or insertable medical devices, which are adapted to be visible under magnetic resonance imaging (MRI). More particularly, the present invention is directed to medical devices, which are provided with a hydrogel coating adapted to render the medical device visible under MRI; the use of such medical devices in a medical procedure during or after which the position of the medical device can be viewed by MRI; and, the use of such coatings to render medical devices coated therewith visible under MRI.

The document WO 03/045457A discloses a medical device with a hydrogel polymer coating, wherein the hydrogel polymer is adapted to render the device visible under MRI.

The document WO 03/094975 A discloses a medical device, which is visible under MRI, with a chelate having an amino or carboxyl group, a paramagnetic-metal ion coordinated therewith, a first hydrogel encapsulating the resulting couplex, and a cross-linker crosslinking the hydrogel with a polymer.

The document W0 00/50103 A discloses a medical device with a polymer having ionic and non ionic crosslinks and a radiopaque filler.

The document JP01 113059 A discloses a plastic appliance wherein the degree of crosslinking decreases in the direction of thickness from the outside to the inside.

The document EP-A-0963796 discloses two layers of different hydrogels for coating of a medical device, wherein the hydrogels may comprise a contrasting agent.

### SUMMARY OF THE INVENTION

According to the present invention, a medical device according to claim 1 is provided. Examples of hydrogel polymers include polysaccharide and polypeptide hydrogel polymers such as alginic acid, hyaluronic acid, chitin, carboxymethyl cellulose, hydroxypropyl cellulose, collagen, and gelatin, as well as salts and copolymers thereof. Upon insertion or implantation of the medical device into a patient, the hydrogel coating is differentiable from the environment surrounding the hydrogel coating under magnetic resonance imaging.

As the phrase is used herein, "absorption upon hydration" refers to an increase in weight that is observed when a dried hydrogel region is immersed in normal saline at 25°C for 1 minute. A "dried hydrogel region" is a hydrogel region dried for at least 12 hours in air at a temperature of 25°C and at a relative humidity of 50% or less.

Hence, the phrase "the inner region exhibits more absorption upon hydration than does the outer region" means that the increase in weight that is observed for a material that corresponds to the inner region, upon immersion in normal saline at 25°C for I minute, will be greater than the increase in weight that is observed for a material that corresponds to the outer region under the same conditions. A specific illustration is drawn from the Examples below, where the material that corresponds to the inner region (i.e., material comprising hydrogel polymer, covalent crosslinking agent, and contrast agent) exhibits a greater increase in weight upon immersion than the material that corresponds to the outer region (i.e., material comprising hydrogel polymer and ionic crosslinking agent). In general, material corresponding to the inner region will experience an increase in weight that is at least 10% greater than the weight increase observed for material (of the same size and shape) corresponding to the outer region.

In certain beneficial embodiments, the first and second hydrogel polymers are the same, and both the inner and outer regions are crosslinked, in which case the crosslink density of the outer region is typically higher than the crosslink density of the inner region.

According to one specific embodiment of the invention, the outer region comprises an ionic crosslinking agent and the inner region comprises a covalent crosslinking agent. Examples of ionic crosslinking agents include agents comprising a multivalent cation, such as calcium, magnesium, barium, strontium, boron, beryllium, aluminum, iron, copper, cobalt, lead or silver cations. Examples of covalent crosslinking agents include polyfunctional crosslinking agents comprising diazonium, azide, isocyanate, acid chloride, acid anhydride, imino carbonate, amino, carboxyl, epoxy, hydroxyl, aldehyde, carbodimide, or aziridine groups, for instance; a polyfunctional aziridine compound.

Beneficial contrast agents for the practice of the present invention include paramagnetic ions, for instance, gadolinium (III) ions, which are typically provided as part of a paramagnetic ion chelation complex, for instance a chelation complex comprising diethylenetriamine pentaacetic acid (DTPA).

Examples of implantable or insertable medical devices having surfaces which act as a substrate for the above hydrogel coatings include catheters (e.g., coronary catheters, peripheral vascular catheters, and neuro-interventional microcatheters), stents (e.g., endovascular, biliary, tracheal, gastrointestinal, urethral, ureteral, esophageal and coronary stents), embolic coils, endovascular grafts, guide wires, and balloons (e.g., coronary balloons, peripheral vascular balloons, and neurological balloons).

These and other embodiments and advantages of the present invention will become readily apparent from the detailed description and claims to follow.

### DETAILED DESCRIPTION OF THE INVENTION

Various embodiments of the present invention now will be described more fully hereinafter. This invention may, however, be embodied in different forms and should not be construed as limited to the embodiments set forth herein.

It is known to provide coatings of the surfaces of implantable or insertable medical devices. Such coatings may be provided for various purposes including, but not limited to, carrying a therapeutic agent for localized delivery to a target area within the body; providing a lubricious surface to facilitate introduction of the medical device into the patient during an interventional procedure; improving the biocompatibility of the medical device with the surrounding environment; or a combination of these or other purposes. Among the coatings that have been proposed for implantable or insertable medical devices are polymeric materials known as hydrogels.

Hydrogels are generally polymeric materials that are hydrophilic and can absorb, relative to their weight, relatively large amounts of water or other polar molecules, typically from 30 wt% to 50 wt% or more. When provided as a coating on a substrate, for example, the surface of a medical device, the hydrogel polymer can swell in the presence of water to several times its thickness in the absence of the water.

Hydrogels have been disclosed as coatings for implantable or insertable medical devices or as materials for constructing the devices in, for example, U.S. Patent Nos. 6,316,522; 6,261,630; 6,184,266; 6,176,849; 6,096,108; 6,060,534; 5,702,754; 5,693,034; and, 5,304,121, each of which is assigned to Boston Scientific Corporation or SciMed Life Systems, Inc.
Hydrogels, such as those described in the foregoing exemplary U.S. Patents, can be based on synthetic or naturally occurring materials, or a composite thereof; they can be biodegradable or substantially non-biodegradable; and, they can be modified or derivatized in numerous ways to render the hydrogel more suitable for a desired purpose.

For example, hydrogel polymers can be modified by covalently crosslinking with, for example, a polyfunctional crosslinking agent that is reactive with functional groups covalently bonded to the polymer structure. Hydrogel polymers can also be ionically crosslinked with, for example, polyvalent metal ions. Many hydrogel polymers described herein can be both covalently and ionically crosslinked. Crosslinking of a hydrogel polymer can be advantageous, for example, to provide a more rigid material, to render the hydrogel less soluble in a particular environment, to modify the ability of the hydrogel polymer to absorb water, or to modify the manner in which water or other molecules, compounds or groups are associated with the hydrogel polymer.

As noted above, MRI based on detection of the spin relaxation of ¹H nuclei (protons) relies on the ability to differentiate protons in an area of interest relative to those in a surrounding environment. For example, protons in the area of interest, while detectable using MRI, are often not sufficiently differentiated from protons surrounding the area of interest to provide the contrast necessary for producing a good image using MRI. However, by changing the magnetic environment of the protons in the area of interest relative to that of protons in the surrounding environment, enhanced detection and contrast can be achieved. For example, it is generally known to decrease the spin relaxation time of protons by incorporating a paramagnetic species such as gadolinium(III) in the environment of the water molecules in the area of interest.

In the present invention, a hydrogel coating is adapted to render an implantable or insertable medical device visible under MRI. Hydrogel coatings are especially suitable for differentiating MRI detectable species, for example, protons associated with the hydrogel polymers, from protons in the environment surrounding the device.

Detectable protons can be associated with hydrogel polymer, for example, by providing them within groups, such as pendant groups, that are covalently or ionically bonded to the hydrogel polymer. Or detectable protons may be present in water or other molecules or compounds that are found within or upon the hydrogel polymer. Thus association between groups, molecules or compounds containing detectable protons and the hydrogel polymer can be facilitated by a variety of mechanisms, including Van der Waals forces, hydrogen bonding, ionic bonding, covalent bonding, physical entrapment and combinations of the same. The association of detectible protons with the hydrogel polymer may be enhanced by providing a crosslinked hydrogel region as discussed further below.

As noted above, in one aspect of the present invention, a hydrogel coating is provided on a medical device substrate, which upon insertion or implantation of the medical device into a patient, is differentiable from the environment surrounding the hydrogel coating under magnetic resonance imaging. More particularly, the present inventors have achieved enhanced visibility under magnetic resonance imaging by providing hydrogel coatings having inner and outer regions, wherein the inner region exhibits more absorption upon hydration than does the outer region. Inner and outer regions having these characteristics can be provided using any number of techniques, for instance, (a) by employing different hydrogel polymers within the inner and outer regions, (b) by varying the type of crosslinking between the regions (e.g., by employing covalent crosslinking within the inner region and employing ionic crosslinking within the outer region), (c) by using the same type of crosslinking mechanism, but varying the type of agent between the regions (e.g., by employing a first covalent crosslinking agent within the inner region and employing a second covalent crosslinking agent within the outer region), (d) by varying degree of crosslinking, and hence the crosslink density, between the regions (e.g., by providing a higher concentration of crosslinking agent within the outer region relative to the inner region), or (e) a combination of these techniques.

Without being bound by theory, it is believed that the hydrogel coatings of the present invention are particularly suitable for magnetic resonance imaging, *inter alia,* because they can be adapted to provide a magnetic environment for detectable species associated with the hydrogel coating (e.g., protons in water or in other molecules, compounds or groups associated with the hydrogel polymer) that differs from the magnetic environment of detectable species in the environment surrounding the hydrogel coatings. These differences in magnetic environment translate into differences in spin relaxation times and therefore enhanced visibility under MRI.

Moreover, and again without being bound by theory, it is believed that by providing a hydrogel coating comprising an outer region that exhibits less absorption than an underlying inner region, the outer region effectively forms a shell around the inner region, regulating the diffusion of the detectable species in and out of the inner region. As a result, the detectable species can be excited and detected within the inner region, with little movement occurring during the course of these processes, resulting in enhanced visibility under MRI. In general, the inner, more water absorbent, region preferably swells sufficiently to allow water to associate with the contrast agent and enhance visibility, but not so much that the coating becomes unstable (e.g., swelling to an extent such that the coating readily peels from the underlying substrate).

Hydrogel polymers for use in connection with the present invention include, without limitation, the hydrogels disclosed in U.S. Patent Nos. 6,316,522; 6,261,630; 6,184,266; 6,176,849; 6,096,108; 6,060,534; 5,702,754; 5,693,034; and, 5,304,121, mentioned above. Specific examples of hydrogel polymers for use within the hydrogel coatings of the invention include polyacrylates; poly(acrylic acid); poly(methacrylic acid); polyacrylamides; poly(N-alkylacrylamides); polyalkylene oxides; poly(ethylene oxide); poly(propylene oxide); poly(vinyl alcohol); poly(vinyl aromatics); poly(vinylpyrrolidone); poly(ethylene imine); poly(ethylene amine); polyacrylonitrile; poly(vinyl sulfonic acid); polyamides; poly(L-lysine); hydrophilic polyurethanes; maleic anhydride polymers; proteins; collagen; cellulosic polymers; methyl cellulose; carboxymethyl cellulose; dextran; carboxymethyl dextran; modified dextran; alginates; alginic acid; pectinic acid; hyaluronic acid; chitin; pullulan; gelatin; gellan; xanthan; carboxymethyl starch; chondroitin sulfate; guar; starch; and copolymers, mixtures and derivatives thereof.

Various methods of crosslinking hydrogel polymers are known and include (a) covalent crosslinking, for example, with polyfunctional reagents that bridge hydrogel polymer chains by reaction with functional groups along the hydrogel polymer chains and (b) ionic crosslinking, for example, using polyvalent metal ions. Other crosslinking methods, such as crosslinking by exposing the hydrogel polymer to light of an appropriate frequency, may also be employed. Crosslinking with polyfunctional reagents and/or with polyvalent metal ions are among the preferred methods of crosslinking, because the degree of crosslinking can frequently be readily controlled, for example, by varying the amount of polyfunctional crosslinking agent and/or the amount of polyvalent metal ions employed, by varying the type of polyfunctional crosslinking agent or polyvalent metal ions employed, or both. Hydrogel polymers that may be covalently crosslinked do not necessarily form a mutually exclusive group from hydrogel polymers that may be ionically crosslinked. In general, the hydrogel polymers useful in accordance with the present invention may be ionically crosslinked, covalently crosslinked, ionically and covalently crosslinked, or crosslinked by other methods known in the art as well.

A polyfunctional crosslinking agent can be any compound having at least two functional groups that react with functional groups in the hydrogel polymer, for instance, amide or organic acid functional groups within the hydrogel polymer. Any conventional polyfunctional crosslinking agent known in the art may be employed. In many embodiments, the crosslinking agent contains one or more ofcarboxyl, hydroxy, epoxy, halogen or amino functional groups which are capable, via well-known mechanisms such as nucleophilic or condensation reactions, of reacting with functional groups present along the hydrogel polymer backbone or otherwise in the hydrogel polymer structure. The polyfunctional crosslinking agent may thus comprise, for example, diazonium, azide isocyanate, acid chloride, acid anhydride, imino carbonate, amino, carboxyl, epoxy, hydroxyl, aldehyde, carbodimide and aziridine groups. Examples of crosslinking agents include polycarboxylic acids and anhydrides; polyamines; epihalohydrins; diepoxides; dialdehydes such as glutaraldehye; diols; carboxylic acid halides, ketenes and like compounds. Examples of crosslinking agents are found in U.S. Patent Nos. 5,869,129, 5,702,754 and 6,060,534. Specific crosslinking agents that may be used in the practice of the present invention include, for example, commercially available preparations sold by Zeneca Resins (e.g., NeoCryl CX 100), preparations sold by EIT Industries (e.g., XAMA-7), and preparations sold by Union Carbide (e.g., Ucarlink XL-29SE). A combination of polyfunctional crosslinking agents may also be used.

Polyfunctional crosslinking agents can be supplied to a coating layer comprising a hydrogel polymer or a hydrogel-polymer-forming monomer, for example, by co-establishing the polyfunctional crosslinking agent with the coating layer or by providing the polyfunctional crosslinking agent in a previously or subsequently established layer.

Among crosslinkable hydrogel polymers useful in the present invention are also polymers characterized by the presence therein of organic acid functional groups that are reactive with polyfunctional crosslinking agents. By "organic acid functional group" is meant any organic group containing an acidic hydrogen atom such as a carboxylic, sulfonic or phosphoric acid group or a metal salt of any such acid group, particularly alkali metal salts of such acid groups such as lithium, sodium and potassium salts, and quaternary amine salts such as quaternary ammonium salts. It is noted, however, that hydrogel polymers which are crosslinked by means other than by reaction of an organic acid functional group of the hydrogel polymer with a polyfunctional crosslinking agent are also included within the scope of the present invention.

Crosslinking ions that are used to ionically crosslink the hydrogel polymers may be anions or cations, depending on whether the polymer is anionically or cationically crosslinkable. Appropriate crosslinking ions include but are not limited to polyvalent cations selected from the group consisting of calcium, magnesium, barium, strontium, boron, beryllium, aluminum, iron, copper, cobalt, lead and silver cations ions. Crosslinking anions may be selected from, but are not limited to, the group consisting of phosphate, citrate, borate, succinate, maleate, adipate and oxalate anions. More broadly, crosslinking anions are commonly derived from polybasic organic or inorganic acids. Ionic crosslinking may be carried out by methods known in the art, for example, by contacting the hydrogel polymers with an aqueous solution containing dissolved ions.

Examples of ionically crosslinkable hydrogel polymers that may be used in accordance with the present invention are disclosed, for example, in U.S. Patent Nos. 6,096,018 and 6,060,534. Ionically crosslinkable polymers can be either cationic or anionic in nature and include carboxylic, sulfate, and amine functionalized polymers such as polyacrylic acid, polymethacrylic acid, polyhydroxy ethyl methacrylate, polyvinyl alcohol, polyacrylamide, poly (N-vinyl pyrrolidone), polyethylene oxide, hydrolyzed polyacrylonitrile, polyethylene amine, polysaccharides, alginic acid, pectinic acid, carboxy methyl cellulose, hyaluronic acid, heparin, heparin sulfate, chitosan, carboxymethyl chitosan, chitin, carboxymethyl starch, dextran, carboxymethyl dextran, chondroitin sulfate, cationic guar, cationic starch, alginic acid, pectinic acid, pullulan, gellan, xanthan, and collagen as well as mixtures, derivatives (such as salts and esters) and copolymers thereof. Many of these polymers are also crosslinkable using covalent crosslinking agents as well, and thus may be crosslinked with covalent crosslinking agents, with ionic crosslinking agents, or both.

Molecules other than water, or in addition to water, may also be associated with the hydrogel polymer to enhance visibility under MRI. Such molecules comprise species, such as protons, that are detectable using MRI when associated with the hydrogel polymer. Examples of such molecules include hydroxyl-group containing compounds such as alkanols, e.g., ethanol, glycerin (glycerol), ethylene glycol, propylene glycol, polyethylene glycol, polypropylene glycol and other hydroxylated and polyhydroxylated compounds which are known in art. When such hydroxyl-group containing compounds are associated with the hydrogel polymer, the protons in the hydroxyl groups may be further differentiated from protons in the environment surrounding the implantable or insertable medical device.

Exemplary contrast agents include paramagnetic materials such as paramagnetic ions, paramagnetic ion chelation complexes, paramagnetic particles and other materials that enhance the visibility under MRI of detectable species such as protons in water or other molecules, compounds or groups that are associated with the hydrogel polymer. Paramagnetic materials are generally believed to function by reducing the spin relaxation time of such detectable species, thereby differentiating such species from detectable species in the environment surrounding the implantable or insertable medical device and, consequently providing the detectable species associated with the hydrogel polymer with enhanced visibility under MRI. Any material known in the art as an MRI contrast agent may be incorporated within the hydrogel coating in accordance with the present invention. In general, at least the lower, more water absorbent, region of the hydrogel coating of the present invention is preferably adapted by incorporating an MRI contrast agent.

Paramagnetic materials are typically those that have a strong magnetic moment relative to detectable protons in water or in other molecules, compounds or groups in the vicinity of the paramagnetic materials. Elements with atomic numbers 21-29, 42, 44, and 58-70, such as chromium (III), manganese (II), iron (III), iron (II), cobalt (II), copper (II), nickel (II), praseodymium (III), neodymium (III), samarium (III), ytterbium (III), gadolinium (III), terbium (III), dysprosium (III), holmium (III) and erbium (III) are examples of paramagnetic elements that can be used in accordance with the present invention. A widely used element in paramagnetic materials for MRI contrast agents is gadolinium (III), a lanthanide having seven unpaired electrons in the 4f orbital, which has a large magnetic moment. The large magnetic moment of the gadolinium (III) is believed to causes a localized reduction of the relaxation times of protons in its environment, resulting in enhanced visibility of the magnetic resonance images. Paramagnetic materials based on gadolinium (III) ions are, therefore, among preferred paramagnetic materials that can be incorporated within a hydrogel polymer coating on an implantable or insertable medical device in accordance with the present invention.

Paramagnetic ions can be incorporated within the hydrogel coating by, for example, contacting a previously formed hydrogel coating with the paramagnetic ions, e.g., a solution of a soluble salt of the paramagnetic ion, or a solution comprising a paramagnetic ion chelation complex. Alternatively, the paramagnetic ions can be incorporated within the hydrogel coating concurrent with its formation, for example, by contacting the hydrogel polymer or a monomer precursor thereof with the paramagnetic ions or a chelation complex thereof prior to formation of the hydrogel coating. Additional techniques for incorporating paramagnetic ions into the hydrogel coatings of the present invention are discussed below.

Where paramagnetic ions are used in the hydrogel coating, it is particularly advantageous if the paramagnetic ions are secured onto and/or within the hydrogel polymer, thereby preventing excessive loss of such ions. Substantial immobilization of the paramagnetic ions is, of course, important to ensure that the hydrogel coating, and hence medical device coated therewith, will have maximum visibility and durability under MRI. Moreover, the paramagnetic ions are often toxic and can, therefore, produce adverse reactions if excessively leached from the coating and thereafter distributed systemically or absorbed in specific areas of the patient.

Paramagnetic ions can be secured by a number of mechanisms. For example, the hydrogel polymer itself can comprise groups that facilitate securing the paramagnetic ions onto and/or within the hydrogel polymer. Such groups may, for example, comprise organic acid functional groups or other anionically ionizable groups in or covalently bonded to the hydrogel polymer (e.g., pendant from the hydrogel polymer backbone). It is believed that the electrostatic attraction or ionic bonding of the cationic paramagnetic ions to such anionic groups secures the paramagnetic ions within and/or onto the hydrogel polymer. The anionically ionizable groups may also be functionally referred to herein as paramagnetic ion chelating groups.

For example, paramagnetic ion chelating groups can be incorporated within a hydrogel polymer by reacting a polymerizable monomer containing a paramagnetic ion chelating functionality with a polymerizable hydrogel monomer. For example, the paramagnetic chelating functionality can be provided by utilizing a polymerizable monomer containing an aminopolycarboxylic acid group. Among aminopolycarboxylic acid groups or other metal chelating groups that may be incorporated within a hydrogel polymer in accordance with the present invention, are those known in the art for chelating metal ions, including diethylene triaminepentaacetic acid (DTPA); 1,4,7,10-tetraazacyclododecane-N,N,N',N'''-tetraacetic acid (DOTA); ethylenediaminetetraacetic acid (EDTA); 1,4,7,10-tetraazacyclododecane-N,N',N"-triacetic acid (DO3A); 1,4,7-triazacyclononane-N,N',N"-triacetic acid (NOTA); 1,4,8,11-tetraazacyclotetradecane-N,N',N",N"'-tetraacetic acid (TETA); and hydroxybenzylethylene-diamine diacetic acid (HBED). The paramagnetic ions may be incorporated within the hydrogel polymer for chelation with such metal chelating groups, for example, before or after the hydrogel polymer is provided on the surface of the implantable or insertable medical device.

The hydrogel polymer used to coat medical devices in accordance with the present invention may also comprise a paramagnetic ion chelation complex that is not covalently incorporated into the polymer chain. Such paramagnetic chelation complexes may, for example, be complexes of any paramagnetic ion, such as those mentioned hereinabove, with any conventional metal chelating compound including, without limitation, DTPA, DOTA, EDTA, NOTA, TETA and HBED. Gadolinium diethylene triaminepentaacetic acid (Gd-DTPA) is among presently preferred paramagnetic ion chelation complexes that can be incorporated in a hydrogel polymer for coating a medical device in accordance with the present invention.

Paramagnetic ion chelation complexes can be non-covalently incorporated within hydrogel coatings in a number of ways. For example, a chelating compound can be incorporated within a hydrogel coating by forming a suspension or dispersion of the hydrogel polymer and chelating compound and, thereafter applying the suspension or dispersion to the surface of a medical device. A chelating compound can also be combined with hydrogel-forming monomers in a suspension or dispersion and thereafter applying the suspension or dispersion to the surface of a medical device, followed by polymerization of the hydrogel monomers. It is also possible to apply the chelating compound to the surface of a medical device previously coated with a hydrogel polymer by, e.g., by spraying a solution of the chelating compound on the polymer, or dipping the polymer into the same.

As previously noted, crosslinked hydrogel polymers are utilized in various embodiments of the present invention. In addition to the previously mentioned advantages of using crosslinked hydrogel polymers in connection with the present invention, crosslinking can also facilitate immobilization of the paramagnetic ions or other paramagnetic materials within the hydrogel coating. For example, where the paramagnetic ions are ionically bonded to an organic acid functionality or metal chelating functionality provided by the hydrogel polymer as described above, immobilization of the paramagnetic ions is further enhanced in connection with a crosslinked hydrogel.

As another example, a polyfunctional crosslinking agent can be used, which further comprises a metal chelating group covalently bonded to the polyfunctional crosslinking agent. Any metal chelating group including, without limitation, DTPA, DOTA, EDTA, NOTA, TETA and HBED, that can be covalently bonded to a crosslinking agent, while allowing the polyfunctional crosslinking agent to crosslink the hydrogel polymer, is within the scope of the present invention. Further, polyfunctional crosslinking agents having, for example, groups such as carboxyl groups that facilitate electrostatic attraction or ionic bonding thereto of paramagnetic cations are within the scope of the present invention. Chelation of paramagnetic ions to chelating groups covalently bonded to the crosslinking agent or electrostatic attraction/ionic bonding of paramagnetic ions to groups covalently bonded to the crosslinking agent may further enhance the ability of the crosslinked hydrogel polymer to immobilize the paramagnetic ions.

As yet another example, the metal chelating compound can also be immobilized by combining a polyfunctional crosslinking agent, a metal chelating compound containing one or more functional groups reactive with the crosslinking agent, and a hydrogel polymer containing one or more functional groups reactive with the crosslinking agent. Any metal chelating group including, without limitation, DTPA, DOTA, EDTA, NOTA, TETA and HBED, that can be covalently bonded to a functional group of a polyfunctional crosslinking agent is within the scope of the present invention. Chelation of paramagnetic ions to chelating groups covalently bonded to the hydrogel by means of a polyfunctional crosslinking agent may further enhance the ability of the crosslinked hydrogel polymer to immobilize the paramagnetic ions.

The hydrogel polymer portion may also have incorporated therein paramagnetic particles. Paramagnetic particles are distinguished herein from paramagnetic ions or chelation complexes of paramagnetic ions in that such particles, as the name implies, are solids. Such solids are preferably substantially insoluble in an aqueous environment, for example, the aqueous environment of a hydrogel having water associated therewith.

Such paramagnetic particles can be incorporated within a hydrogel polymer by, for example, forming a suspension or dispersion containing the hydrogel polymer and paramagnetic particles and, thereafter applying the suspension or dispersion to the surface of a medical device, followed by drying. The paramagnetic particles can also be combined with the hydrogel-forming monomers prior to polymerizing the same. It is also possible to apply the paramagnetic particles to the surface of a medical device previously coated with a hydrogel polymer by, e.g. contacting the coated medical device with the particles by spraying the particles onto the coated medical device. Other methods known in the art for coating polymeric surfaces with particulate substances may also be adapted to provide a hydrogel coated medical device in accordance with the present invention wherein a surface thereof comprises paramagnetic particles. As above, crosslinking commonly enhances the degree to which the paramagnetic particles are secured to the hydrogel coating.

Any paramagnetic particles known in the art for use as MRI contrast agents may be utilized in this embodiment of the present invention. Among such paramagnetic particles that are useful, therefore, include solid compounds of any of the paramagnetic elements mentioned above. Preferred solid compounds of such elements include the paramagnetic and superparamagnetic oxides of such elements. Among presently preferred paramagnetic particles are ultra-small superparamagnetic iron oxide particles coated with starch or other polysaccharides or cellulosic materials. Examples of such coated ultra-small iron oxide particles are found, for example, in U.S. Patent No. 6,207,134 and 6,123,920 (and the patents cited therein) assigned to Nycomed Imaging AS.

A hydrogel coating may be provided on a substrate surface (i.e., a medical device surface) by a variety of techniques known in the art. For example, a hydrogel coating may be provided by dipping the medical device, or portion thereof to be coated with the hydrogel polymer, into a solution, dispersion or emulsion containing the hydrogel polymer, as well as any other optional reagents such as crosslinking agents, followed by drying to remove the carrier fluid used to dissolve, disperse or emulsify the hydrogel polymer. A solution, dispersion or emulsion of the hydrogel polymer may also be sprayed onto the surface of the medical device followed by drying.

Other methods known in the art for providing a hydrogel coating on the surface of a substrate may also utilized. For example, a hydrogel polymer may be polymerized on the surface of the medical device by contacting the medical device or portion thereof with a solution, dispersion or emulsion containing a polymerizable monomer, as well as any other optional reagents such as crosslinking agents, initiators, etc., and thereafter causing polymerization to occur *in situ* on the surface of the medical device. The polymerizable monomer may also be deposited on the surface of the medical device by, for example, plasma enhanced chemical vapor deposition (PECVD) or other methods known in the art. The polymerization reaction occurring on the surface of the medical device may be triggered by, for example, heating the coated medical device or exposing the coated medical device to light of an appropriate wavelength. Other methods known in the art for causing polymerization to occur on a substrate may be utilized to provide a hydrogel polymer on the surface of an implantable or insertable medical device in accordance with the present invention.

The surface of the medical device may be pre-treated or primed to enhance adherence of the hydrogel polymer or polymerizable monomer to the surface thereof. Such pre-treatment typically results in a hydrogel polymer coating that is more tenaciously adhered to the medical device.

For example, a coating of primer solution is applied to the device surface through conventional methods, for example, dipping or spraying. The primer solution may comprise, for example, a solution, dispersion or emulsion of a primer polymer and an excess of a polyfunctional crosslinking agent that is reactive with functional groups on the primer polymer and on as well as functional groups on a hydrogel polymer that is subsequently applied to the medical device. After the primer solution is permitted to dry to form a substantially water-insoluble layer, a solution, dispersion or emulsion of a hydrogel polymer or hydrogel-forming monomers is applied by conventional methods. The solution, dispersion or emulsion of hydrogel polymer or hydrogel-forming monomer may further comprise, as needed, optional initiator, crosslinking agent, or paramagnetic material such as a paramagnetic ion, paramagnetic ion chelation complex or a paramagnetic particle. The hydrogel polymer is bonded to the primer coating layer through excess unreacted polyfunctional crosslinking agent in the primer coating.

One method for attaching the hydrogel polymer to the surface of a medical device that may be advantageously employed is disclosed in U.S. Patent No. 5,702,752. In this method, the substrate, i.e., the surface of a medical device, is coated with a primer coating composition comprising, for example, an aqueous dispersion or emulsion of a polymer having organic acid functional groups and a polyfunctional crosslinking agent having functional groups capable of reacting with the organic acid functional groups in the subsequently applied hydrogel polymer. The primer coating layer is dried to form a substantially insoluble layer and then the substrate having the primer coating layer disposed thereon is contacted with a solution, dispersion or emulsion of a hydrogel polymer. The polyfunctional crosslinking agent used in this method provides unreacted functional groups for reaction with functional groups, such as organic acid functional groups, in the hydrogel polymer. The functional groups in the polyfunctional crosslinking agent thus serve at least two purposes. The first purpose is to crosslink the primer coating and thereby form a substantially water insoluble primer coating layer. The second purpose is to covalently bond to organic acid groups present in the hydrogel polymer, thereby securing the hydrogel polymer to the primer coating layer. Therefore, sufficient functionality is preferably present in the crosslinking agent to accomplish both purposes. That is, the amount/type of crosslinking agent used is preferably sufficient such that enough functional groups are present to substantially crosslink the primer coating and provide unreacted functional groups for covalently bonding to the hydrogel polymer.

Unreacted functional groups in the polyfunctional crosslinking agent may be present, for example, by supplying an excess of the polyfunctional crosslinking agent during application of the primer coating and/or by utilizing a polyfunctional crosslinking agent having more than two functional groups per molecule. Among such polyfunctional crosslinking agents having more than two functional groups per molecule are trifunctional aziridines disclosed in U.S. Patent No. 5,702,754.

In various embodiments of the present invention, the inner hydrogel region, the outer hydrogel region or the entire hydrogel coating is provided with an optional osmotic agent, which promotes hydration (and hence swelling) of the coating by creating an osmotic gradient between the coating and an aqueous media into which the coating is immersed. Examples of osmotic agents include various pharmaceutically acceptable sugars and salts, such as sodium chloride, potassium chloride, and calcium chloride. For example, in the Examples below, sodium chloride is used for this purpose.

In various embodiments of the present invention, the inner hydrogel region, the outer hydrogel region or the entire hydrogel coating is provided with an optional plasticizer to soften the hydrogel coating. Examples of plasticizers include glycerol, dextran and polysorbitol. For example, in the Examples below, glycerol is used for this purpose.

Finally an optional lubricious coating layer may be provided by methods similar to those set forth above. This additional coating layer preferably comprises a hydrogel polymer that provides lubricity, which can facilitate insertion of the implantable or medical device into the patient.

The hydrogel coatings of the present invention, when provided on a surface of a medical device in accordance with the present invention, will preferably have an overall thickness in the range of from 20 to 3000 microns, more preferably from 50 to 2000 microns. Hydrogel coating thicknesses in the range of from 100 to 1000 microns when hydrated are particularly preferred. The thickness of the hydrogel coating may be adjusted to enhance the visibility under MRI of the medical device, or a portion thereof, coated with a hydrogel polymer in accordance with the present invention. For example, the medical device may be provided with a substantially uniform thickness of the hydrogel polymer, or selected portions of the medical device may be provided with a thicker or thinner hydrogel polymer coating as desired to enhance contrast with respect to another portion of the medical device. It is understood that the entire surface of the medical device need not be provided with a hydrogel coating in accordance with the present invention. Thus, the coating may be provided only on selected portions of the medical device to enhance the visibility thereof or to render such portions visible under MRI.

The present invention has wide applicability to all types of implantable or insertable medical devices known in the art whether such medical devices are used primarily in conjunction with observational, diagnostic or therapeutic medical procedures. Most generally, the present invention may be practiced with any implantable or insertable medical device for which visualization thereof under MRI during or after insertion of the device is desired.

Among such implantable or insertable medical devices are included, without limitation, catheters such as neuro-interventional microcatheters; guide wires; balloons such as those used in angioplasty procedures; stents including endovascular, biliary, tracheal, gastrointestinal, urethral, ureteral and esophageal stents; stent grafts; prosthetic devices such as artificial limbs; endoscopic devices; and laparoscopic devices. The medical device comprising the substrate over which the hydrogel, coating is provided in accordance with the present invention can be constructed of any material conventionally used for such devices including metals and metal alloys such as superelastic shape memory alloys; ceramics; glasses; polymeric materials which may be based on natural, semi-synthetic, and synthetic polymeric materials; and composites of any of such materials. The material is preferably a biocompatible material, i.e., does not produce, either systemically or locally, an unacceptable adverse reaction in the patient, and may be biodegradable or substantially non-biodegradable in the environment surrounding the medical device upon insertion or implantation thereof. Biocompatibility of the material used to construct the medical device may be enhanced by providing a biocompatible hydrogel coating in accordance with the present invention.

The hydrogel polymer coating the medical device in accordance with the present invention can also be adapted to incorporate a diagnostic or a therapeutic agent such as a drug. Incorporation of a therapeutic agent in a coating provided on the surface of a medical device is generally known in the art and is advantageous *inter alia,* because it enables localized administration of the therapeutic agent. Localized administration is, in many cases, beneficial to ensure that a therapeutically effective amount of the agent is administered to the target location and to minimize adverse reactions associated with systemic administration of the agent. Any methods known in the art for loading a therapeutic agent within or on the surface of a polymeric material to be provided as a coating on a medical device can be employed. In the context of the present invention, the therapeutic agent can be included within a solution, dispersion or emulsion of the hydrogel polymer or hydrogel-forming monomer to be provided as a coating onto the medical device. Alternatively, the therapeutic agent can be incorporated into a hydrogel coating previously applied onto the medical device by, for example, dipping or soaking the medical device in a solution or dispersion of the therapeutic agent, or by spraying a solution or dispersion of the therapeutic agent onto the medical device coated with the hydrogel polymer.

### EXAMPLES

The following are examples directed to methods of preparing and testing various hydrogel coatings. These examples should not be construed as limiting the scope of the invention in any manner.

### EXAMPLE 1

### Preparation of Primer Solutions

### 2% Crosslink Primer Solution

In a glass beaker, 980 grams of Bayhydrol PR240, available through Bayer, and 20 grams of NeoCryl CX-100, available through NeoResin, were stirred until thoroughly mixed. Bayhydrol PR240 is a solvent-free anionic aliphatic polyurethane dispersion in water. NeoCryl CX-100 is a polyfunctional aziridine crosslinking agent.

### 4% Crosslink Primer Solution

In a glass beaker, 960 grams of Bayhydrol PR240, available through Bayer, and 40 grams of NeoCryl CX-100, available through NeoResin, were stirred until thoroughly mixed.

### 6% Crosslink Primer Solution

In a glass beaker, 940 grams of Bayhydrol PR240, available through Bayer, and 60 grams of NeoCryl CX-100, available through NeoResin, were stirred until thoroughly mixed.

### EXAMPLE 2

### Preparation of Inner Hydrogel Solutions

### 0.1% Crosslink Inner Hydrogel Solution

949 grams of deionized water were poured into a glass beaker. 30 grams sodium alginate (Protanal LF 10/60, G/M=65-75/25-35, from FMC Biopolymer) was slowly added into the beaker with stirring, which continued until the alginate is dissolved. 10 grams of Gd-DTPA, from Aldrich, was then added into the alginate solution very slowly, with stirring, to avoid forming a precipitate. At the same time ammonium hydroxide was added dropwisely to adjust the pH to around 9 and promote the dissolving of Gd-DTPA. 10 grams of sodium chloride solution were then added with stirring until dissolved. Preparation was complete after 1 gram of NeoCryl CX-100 was added dropwise with agitation.

### 0.25% Crosslink Inner Hydrogel Solution

A solution is prepared as above, except that 947.5 g of deionized water and 2.5g of NeoCryl CX-100 were used.

### 0.35% Crosslink Inner Hydrogel Solution

A solution is prepared as above, except that 946.5 g of deionized water and 3.5g of NeoCryl CX-100 were used.

### 0.50% Crosslink Inner Hydrogel Solution

A solution is prepared as above, except that 945 g of deionized water and 5g of NeoCryl CX-100 were used.

### 1.0% Crosslink Inner Hydrogel Solution

A solution is prepared as above, except that 940 g of deionized water and 10g of NeoCryl CX-100 were used.

### EXAMPLE 3

### Preparation of Outer Hydrogel Solution

940 grams of deionized water were poured into a glass beaker. 30 grams of sodium alginate (Protanal LF 10/60, G/M=65-75/25-35, from FMC Biopolymer) were slowly added into the beaker with stirring, which continued until the alginate was dissolved. 10 grams of sodium chloride solution was then added with stirring until dissolved. Preparation was complete after 20g of glycerol were added with stirring.

### EXAMPLE 4

### Preparation of Hydrogel Coatings

A 2.3 French Renegade® microcatheter was immersed into Primer Solution and air dried. The catheter was then immersed into Inner Hydrogel Solution and dried in the open air. This procedure was repeated 4 times to build up layer thickness. The catheter was then immersed into Outer Hydrogel Solution and air dried. Finally, in some cases, the catheter was sprayed with calcium chloride solution (30% calcium chloride dehydrate in water) and dried. The above process was carried out for various Primer Solutions in combination with various Inner Hydrogel Solutions.

### EXAMPLE 5

### MRI Images of the Hydrogel Coatings

The hydrogel samples coated in Example 4 were evaluated under a 2 Tesla GE MRI machine. Imaging sequence parameters are as follows: Spoiled GRASS with TR/TE=17/4.6ms; FA=30°; matrix=256x256; TA=1'1sec; FOV=12cm; slide thickness=3mm. After the coated samples were soaked in saline for 5 minutes, the image was taken in a saline phantom. Mechanical properties were evaluated after soaking in saline for 1-4 hours. The results are presented in the following table:

| **CX % in Inner Hydrogel Solution** | **Outer coat Ca²⁺ Spray** | **CX % in Primer Solution** | | | | | |
|---|---|---|---|---|---|---|---|
| | | **2.0%** | | **4.0%** | | **6.0%** | |
| | | **Imaging** | **Swelling** | **Imaging** | **Swelling** | **Imaging** | **Swelling** |
| 0.10% | Ca²⁺ | Visible | Swelling | Visible | Little | Visible | Little |
| 0.25% | Ca²⁺ | Visible | Swelling | Visible | Little | Visible | Very little |
| | No Ca²⁺ | Not Visible | Very much | | | Not Visible | Very much |
| 0.35% | Ca²⁺ | Not visible | Little | Visible | Very little | Visible | Very little |
| | No Ca²⁺ | | | Not visible | Very much | | |
| 0.50% | Ca²⁺ | | | Not visible | Very little | | |
| 1.00% | Ca²⁺ | | | Not Visible | Very little | | |

Thus, the combination of Primer Solution having 4.0% or 6.0% CX-100 with Inner Hydrogel Solution having 0.10%, 0.25% and 0.35% CX-100 yielded catheters having both visible magnetic resonance imaging and having little or very little swelling. The other formulations were either not visible under magnetic resonance imaging or exhibited excessive swelling. In general, it has been found that the coating should exhibit enough swelling to allow sufficient water to associate with the Gd agent, which is required to render the coating visible under magnetic resonance imaging. However, if the swelling is too great, the coating becomes unstable and readily peels off of the device.

## Claims

1. A medical device comprising:
(a) a substrate having a surface; and
(b) a hydrogel coating disposed over at least a portion of the substrate surface and comprising inner and outer regions, said inner region exhibiting more absorption upon hydration than does said outer region, said inner region comprising a first hydrogel polymer and said outer region comprising a second hydrogel polymer, said first and second hydrogel polymers being the same, wherein the crosslink density of the second hydrogel polymer is higher than the crosslink density of the first hydrogel polymer, or different, and said hydrogel coating further comprising a contrast agent,
wherein said hydrogel coating is differentiated from the environment surrounding the hydrogel coating under magnetic resonance imaging upon insertion or implantation of said medical device into a patient.

2. The medical device of claim 1, wherein said first and second hydrogel polymers are the same.

3. The medical device of claim 1, wherein said first and second hydrogel polymers are different.

4. The medical device of claim 2, wherein said first and second hydrogel polymers comprise polysaccharide or polypeptide chains.

5. The medical device of claim 3, wherein said first and second hydrogel polymers comprise polysaccharide or polypeptide chains.

6. The medical device of claim 2, wherein said first and second hydrogel polymers are selected from alginic acid, hyaluronic acid, acrylic acid, methacrylic acid, chitin, chitosan, carboxymethyl chitosan, carboxymethyl cellulose, hydroxypropyl cellulose, collagen, gelatin, poly(hydroxy ethyl methacrylate), polyvinyl alcohol, polyacrylamide, poly(N-vinyl pyrrolidone), polyethylene oxide; hydrolyzed polyacrylonitrile, polyethylene amine, heparin, heparin sulfate, dextran, carboxymethyl dextran, chondroitin sulfate, cationic guar, cationic starch, carboxymethyl starch, gellan, xanthan, and salts and copolymers thereof.

7. The medical device of claim 3, wherein said first and second hydrogel polymers are selected from alginic acid, hyaluronic acid, acrylic acid, methacrylic acid, chitin, chitosan, carboxymethyl chitosan, carboxymethyl cellulose, hydroxypropyl cellulose, collagen, gelatin, poly(hydroxy ethyl methacrylate), polyvinyl alcohol, polyacrylamide, poly(N-vinyl pyrrolidone), polyethylene oxide, hydrolyzed polyacrylonitrile, polyethylene amine, heparin, heparin sulfate, dextran, caboxymethyl dextran, chondroitin sulfate, cationic guar, cationic starch, carboxymethyl starch, gellan, xanthan, and salts and copolymers thereof.

8. The medical device of claim 2, wherein said inner and outer regions are each crosslinked.

9. The medical device of claim 3, wherein said inner and outer regions are each crosslinked.

10. The medical device of claim 1, wherein said inner and outer regions are each crosslinked and wherein the crosslink density of said outer region is higher than the crosslink density of said inner region.

## Patentansprüche

1. Medizinische Vorrichtung mit:
(a) einem Substrat mit einer Oberfläche; und
(b) einer Hydrogelbeschichtung, die über mindestens einem Abschnitt der Substratoberfläche angeordnet ist und einen Innen- und einen Außenbereich aufweist, wobei der Innenbereich stärkere Absorption bei Hydratation als der Außenbereich zeigt, der Innenbereich ein erstes Hydrogelpolymer aufweist und der Außenbereich ein zweites Hydrogelpolymer aufweist, wobei das erste und zweite Hydrogelpolymer die gleichen Hydrogelpolymere sind, und die Vernetzungsdichte des zweiten Hydrogelpolymers höher als die Vernetzungsdichte des ersten Hydrogelpolymers ist, oder wobei sie sich unterscheiden, und wobei die Hydrogelbeschichtung ferner ein Kontrastmittel aufweist,
wobei sich die Hydrogelbeschichtung von der Umgebung der Hydrogelbeschichtung unter Magnetresonanztomographie nach Einsetzen oder Implantieren der medizinischen Vorrichtung in einen Patienten unterscheidet.

2. Medizinische Vorrichtung nach Anspruch 1, wobei das erste und zweite Hydrogelpolymer die gleichen Hydrogelpolymere sind.

3. Medizinische Vorrichtung nach Anspruch 1, wobei sich das erste und zweite Hydrogelpolymer unterscheiden.

4. Medizinische Vorrichtung nach Anspruch 2, wobei das erste und zweite Hydrogelpolymer Polysaccharid- oder Polypeptidketten aufweisen.

5. Medizinische Vorrichtung nach Anspruch 3, wobei das erste und zweite Hydrogelpolymer Polysaccharid- oder Polypeptidketten aufweisen.

6. Medizinische Vorrichtung nach Anspruch 2, wobei das erste und zweite Hydrogelpolymer aus Alginsäure, Hyaluronsäure, Acrylsäure, Methacrylsäure, Chitin, Chitosan, Carboxymethylchitosan, Carboxymethylcellulose, Hydroxypropylcellulose, Collagen, Gelatine, Poly(hydroxyethylmethacrylat), Polyvinylalkohol, Polyacrylamid, Poly(N-vinylpyrrolidon), Polyethylenoxid, hydrolysiertem Polyacrylnitril, Polyethylenamin, Heparin, Heparinsulfat, Dextran, Carboxymethyldextran, Chondroitinsulfat, kationischem Guar, kationischer Stärke, Carboxymethylstärke, Gellan, Xanthan sowie deren Salzen und Copolymeren ausgewählt sind.

7. Medizinische Vorrichtung nach Anspruch 3, wobei das erste und zweite Hydrogelpolymer aus Alginsäure, Hyaluronsäure, Acrylsäure, Methacrylsäure, Chitin, Chitosan, Carboxymethylchitosan, Carboxymethylcellulose, Hydroxypropylcellulose, Collagen, Gelatine, Poly(hydroxyethylmethacrylat), Polyvinylalkohol, Polyacrylamid, Poly(N-vinylpyrrolidon), Polyethylenoxid, hydrolysiertem Polyacrylnitril, Polyethylenamin, Heparin, Heparinsulfat, Dextran, Carboxymethyldextran, Chondroitinsulfat, kationischem Guar, kationischer Stärke, Carboxymethylstärke, Gellan, Xanthan sowie deren Salzen und Copolymeren ausgewählt sind.

8. Medizinische Vorrichtung nach Anspruch 2, wobei der Innen- und Außenbereich jeweils vernetzt ist.

9. Medizinische Vorrichtung nach Anspruch 3, wobei der Innen- und Außenbereich jeweils vernetzt ist.

10. Medizinische Vorrichtung nach Anspruch 1, wobei der Innen- und Außenbereich jeweils vernetzt ist und wobei die Vernetzungsdichte des Außenbereichs höher als die Vernetzungsdichte des Innenbereichs ist.

## Revendications

1. Dispositif médical comprenant :
(a) un substrat possédant une surface ; et
(b) un revêtement d'hydrogel disposé sur au moins une partie de la surface du substrat et comprenant des régions interne et externe, ladite région interne présentant une absorption plus importante lors de l'hydratation que ladite région externe, ladite région interne comprenant un premier polymère d'hydrogel et ladite région externe comprenant un second polymère d'hydrogel, lesdits premier et second polymères d'hydrogel étant identiques, dans lequel la densité de réticulation du second polymère d'hydrogel est supérieure à la densité de réticulation du premier polymère d'hydrogel, ou différents, et ledit revêtement d'hydrogel comprenant en outre un agent de contraste,
dans lequel ledit revêtement d'hydrogel se différencie de l'environnement entourant le revêtement d'hydrogel sous imagerie par résonance magnétique lors de l'insertion ou de l'implantation dudit dispositif médical dans un patient.

2. Dispositif médical selon la revendication 1, dans lequel lesdits premier et second polymères d'hydrogel sont identiques.

3. Dispositif médical selon la revendication 1, dans lequel lesdits premier et second polymères d'hydrogel sont différents.

4. Dispositif médical selon la revendication 2, dans lequel lesdits premier et second polymères d'hydrogel comprennent des chaînes de polysaccharides ou de polypeptides.

5. Dispositif médical selon la revendication 3, dans lequel lesdits premier et second polymères d'hydrogel comprennent des chaînes de polysaccharides ou de polypeptides.

6. Dispositif médical selon la revendication 2, dans lequel lesdits premier et second polymères d'hydrogel sont choisis parmi l'acide alginique, l'acide hyaluronique, l'acide acrylique, l'acide méthacrylique, la chitine, le chitosan, le chitosan carboxyméthylé, la carboxyméthylcellulose, l'hydroxypropylcellulose, le collagène, la gélatine, le poly(méthacrylate d'hydroxyéthyle), le poly(alcool vinylique), le polyacrylamide, la poly(N-vinyl pyrrolidone), le poly(oxyde d'éthylène), le polyacrylonitrile hydrolysé, la polyéthylèneamine, l'héparine, le sulfate d'héparine, le dextran, le dextran carboxyméthylé, la chondroïtine-sulfate, le guar cationique, l'amidon cationique, l'amidon carboxyméthylé, la gellane, le xanthane et les sels et copolymères de ceux-ci.

7. Dispositif médical selon la revendication 3, dans lequel lesdits premier et second polymères d'hydrogel sont choisis parmi l'acide alginique, l'acide hyaluronique, l'acide acrylique, l'acide méthacrylique, la chitine, le chitosan, le chitosan carboxyméthylé, la carboxyméthylcellulose, l'hydroxypropylcellulose, le collagène, la gélatine, le poly(méthacrylate d'hydroxyéthyle), le poly(alcool vinylique), le polyacrylamide, la poly(N-vinyl pyrrolidone), le poly(oxyde d'éthylène), le polyacrylonitrile hydrolysé, la polyéthylèneamine, l'héparine, le sulfate d'héparine, le dextran, le dextran carboxyméthylé, la chondroïtine-sulfate, le guar cationique, l'amidon cationique, l'amidon carboxyméthylé, la gellane, le xanthane et les sels et copolymères de ceux-ci.

8. Dispositif médical selon la revendication 2, dans lequel lesdites régions interne et externe sont chacune réticulées.

9. Dispositif médical selon la revendication 3, dans lequel lesdites régions interne et externe sont chacune réticulées.

10. Dispositif médical selon la revendication 1, dans lequel lesdites régions interne et externe sont chacune réticulées et dans lequel la densité de réticulation de ladite région externe est supérieure à la densité de réticulation de ladite région interne.
